# EUROPEAN PATENT APPLICATION

(11) **EP 2 196 456 A1**
(43) Date of publication of application: **16.06.2010**
(21) Application number: 08021530.4
(22) Date of filing: 11.12.2008
(51) Int. Cl.: C07C 303/20, C07C 309/24, C07D 261/14

(54) **A method for preparing sulfur-containing compounds**

(71) Applicant: The Royal College of Surgeons in Ireland, Dublin 2 (IE)
(72) Inventor: Adamo, Mauro, Dublin 2 (IE); Nagabelli, Murali, Dublin 8 (IE)
(74) Representative: O'Connell, Maura

(57) **Abstract**

The invention provides a method for preparing sulfur-containing compounds, the method comprising reacting a donor compound comprising at least one sulfur having at least one lone pair of electrons, with an acceptor compound; wherein the reaction occurs in the presence of a catalyst capable of activating the sulfur having at least one lone pair of electrons.

The invention also provides sulfur-containing compounds of the formula: 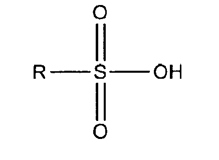 wherein R is selected from:
(a) 1-(4-Nitro-phenyl)-3-oxo-3-phenyl-propane;
(b) 2-(3-Methyl-4-nitro-isoxazol-5-yl)-1-phenyl-ethane;
(c) 1-(4-Methoxy-phenyl)-2-(3-methyl-4-nitro-isoxazol-5-yl)-ethane;
(d) 2-(3-Methyl-4-nitro-isoxazol-5-yl)-1-(4-nitro-phenyl)-ethane;
(e) 1-(4-Fluoro-phenyl)-2-(3-methyl-4-nitro-isoxazol-5-yl)-ethane;
(f) 1-(4-Chloro-phenyl)-2-(3-methyl-4-nitro-isoxazol-5-yl)-ethane;
(g) 3-Oxo-butane;
(h) 2-Methyl-4-oxo-pentane-2-yl;
(i) 3,4-Dihydroxy-naphthalene-2-yl; and
(j) 3-Oxo-cyclohexane.

Finally, the invention provides use of chiral sulfur-containing compounds obtainable by the above-mentioned method or chiral sulfur-containing compounds as mentioned above for the resolution of racemic mixtures of amines.

## Description

This invention relates to methods for preparing sulfur-containing compounds, such as sulfonic acid compounds. Optionally, it relates to methods for preparing heterochiral sulfur-containing compounds, such as heterochiral sulfonic acid compounds, as well as sulfones and sulfoxides, sulfones arising from the addition of sulfinic acids to alkenes and sulfoxides arising from the addition of sulfenic acids to alkenes and alkynes.

### Background to the Invention

Reaction of bisulfite with α,β-unsaturated ketones, esters, and amides has been known for over a century (**Scheme 1**) [(a) Beilstein, F. K. et Al. Chem. Ber. 1885, 18, 482. (b) Dodge, F. D. J. Am. Chem. Soc. 1930, 52, 1724]. 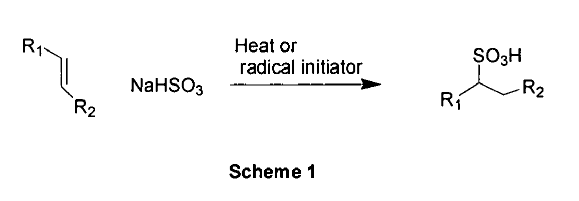 However, there are few reported uses of this reaction. Most examples used highly activated disubstituted enones, require long reaction times, or the use of high temperatures and microwave. Alternatively, a radical initiator is employed in combination with high temperature. Taken together, it is evident that the addition of bisulfite to α,β-unsaturated ketones, esters, and amides occurs only under enforcing conditions and therefore is unpractical. Additionally, the stringency of the required conditions imposes limits on the range of α,β-unsaturated ketones, esters, and amide substrates for use in the reaction.

Sulfonic acids, sulfones and sulfoxides are present in a wide number of marketed compounds. For example, taurine is present is several soft drinks. Presently, the production of sulfonic acids involves the reaction of alkenes and bisulfite at high temperature and/or in the presence of radical initiators. The use of flammable compounds (alkenes) in gas phases at high temperature and/or the use of radical species raise both the risk of explosions and the manufacturing cost. It would be advantageous to prepare sulfonic acids, sulfones and sulfoxides without the need for heating-cooling equipment and without the need for radical chemistry, making the preparation of the sulfur-containing compounds safer, cleaner and more cost efficient.

At present, no enantioselective version of this reaction has been reported. Chiral sulfonic acids are also employed for the resolution of racemic mixtures of amines. When the chiral pool cannot be employed, resolution is the most frequently alternative used by industry to obtain single enantiomers. "Dutch resolution" is a technique for the resolution of racemates that makes use of families of resolving agents. However, there exists only a few families of resolving agents. Commonly used compounds such as quinine, brucine, camphor sulfonic acid, and bromocamphor sulfonic acid tend to be rare. This means that, at present, two steps are required (addition of bisulfite to alkenes and subsequent resolution) to obtain chiral sulfonic acids.

Racemic sulfonic acids, also known as alpha olefin sulfonates (AOS), are a type of anionic surfactant capable of excellent emulsifying, decontaminating and calcium soap dispersing performances. Advantages include good solvency and compatibility, rich and fine foam, easy biodegradation, low toxicity and low irritation to skin. Especially in the application of non-phosphorus detergents, sulfonic acids have not only the good washing ability, but also good compatibility with enzyme agents. Powder (grain) shape products have good fluidity, therefore they are widely used in non-phosphorus washing powder, liquid detergents and home washing products, textile, printing and dyeing industry, petrochemical products, and industrial hard surface cleaning agents.

### Summary of the Invention

According to a first aspect of the present invention, there is provided a method for preparing sulfur-containing compounds, the method comprising reacting
a donor compound comprising at least one sulfur having at least one lone pair of electrons,
with an acceptor compound;
wherein the reaction occurs in the presence of a catalyst capable of activating the sulfur having at least one lone pair of electrons.

The reaction may be carried out using any suitable procedure, wherein the donor compound, the acceptor compound, and the catalyst are reacted under suitable conditions to provide the required sulfur-containing compounds.

By the term "sulfur-containing compounds" is meant an acid comprising at least one sulfur atom, and esters thereof or amides thereof, as well as sulfones and sulfoxides. Optionally, the sulfur-containing compound is an organic compound. Non-limiting examples of sulfur-containing compounds include sulfonic acids, sulfinic acids, sulfenic acids, and esters thereof or amides thereof.

Advantageously, the reaction occurs without the requirement of providing a radical initiator. By "radical initiator" is meant a substance capable of producing a radical species, preferably under mild reaction conditions. More specifically, the reaction occurs in the presence of less than 0.001% (g/g) radical initiator. Typical radical initiators include hydrogen peroxide, benzoyl peroxide, tert-butylperoxide, samarium iodide, cerium ammonium nitrate and their combination with light and heating. Preferably, the reaction occurs in the presence of no radical initiator. The absence of radical initiators is supported by the reaction proceeding in the presence of radical scavengers and at room temperature, as is exemplified hereunder.

Optionally or additionally, the reaction occurs in the presence of a reaction solvent. Optionally, the reaction solvent is inert.

Optionally, the reaction solvent is aprotic. It will be appreciated that aprotic solvents cannot donate hydrogen. Polar aprotic solvents are solvents that share ion dissolving power with protic solvents but lack an acidic hydrogen. Polar aprotic solvents generally have high dielectric constants and high polarity. Examples are dimethyl sulfoxide, dimethylformamide, dioxane and hexamethylphosphorotriamide and tetrahydrofuran. Further optionally, the reaction solvent is tetrahydrofuran.

Alternatively, the reaction solvent is protic, of which methanol is preferred.

Further optionally, the reaction could be carried out in a biphasic system, in which, for example, the donor compound is in one phase and the acceptor compound is in another phase. One such biphasic system is water / toluene and one such biphasic catalyst might be an ammonium salt, for example Bu₄NOH. For example, the donor compound might be in the aqueous phase and the acceptor compound in the toluene phase.

### Donor Compound

The term "a donor compound comprising at least one sulfur having at least one lone pair of electrons" is intended to include bisulfite, sulfinic and sulfenic acids. R groups, when present, could be any alkyl or aryl or heteroaryl group: 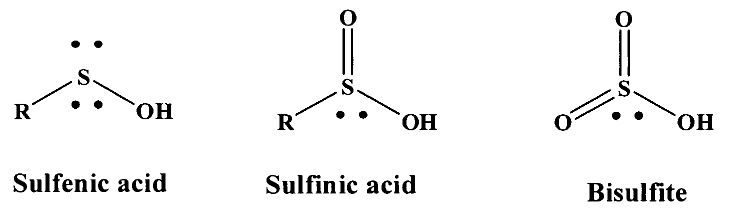 Optionally, the at least one sulfur having at least one lone pair of electrons is capable of being transferred to the acceptor compound. The sulfur having at least one lone pair of electrons can be transferred via the formation of an intermediate compound, or may be directly transferred to the acceptor compound.

Preferably, the at least one sulfur having at least one lone pair of electrons is a sulfite anion.

Preferably, the donor compound is sodium hydrogen sulfite (sodium bisulfite).

### Acceptor Compound

By the term "acceptor compound" is meant an unsaturated compound or a cyclic derivative thereof. For example, a cyclic derivative of an alkene comprises an epoxide or an aziridine.

By the term "unsaturated compound" is meant a polyatomic compound, wherein the bond order between at least one pair of atoms is greater than 1. For the purposes of this specification, an unsaturated compound is intended to include any compound having a double-, triple-, or higher order-bond between at least one pair of atoms. The term "multiple bond" is intended to include any double-, triple-, or higher order-, bond. Non-limiting examples include an alkene or an alkyne. The at least one multiple bond extends between two adjacent atoms that may be the same atom or different atoms. Preferably, at least one atom is a carbon atom. Further preferably, both atoms are carbon atoms.

When the acceptor compound is an unsaturated compound, at least one electron-withdrawing group is adjacent the at least one multiple bond. By "electron-withdrawing group" is meant any group that causes uneven electron distribution within the at least one multiple bond. Such "electron-withdrawing groups" include, but are not limited to, carbonyl groups, and their esters; nitro groups; cyano groups; oximes; hydrazones; imino groups; protected imino groups such as BOC or CBZ protected imines.

Where the acceptor compound is a cyclic derivative of an unsaturated compound, it is not thought necessary to have at least one electron-withdrawing group adjacent the cyclic derivative. Without being bound by theory, it is thought that the electrons within the cyclic derivative are already unevenly distributed, making the presence of an adjacent electron-withdrawing group unnecessary.

Optionally, the unsaturated compound may be linear or branched. Preferably, the unsaturated compound is an alkene. Alternatively, the unsaturated compound is an arene.

Alternatively, the unsaturated compound may be cyclic. The unsaturated compound may be monocyclic, polycyclic, or heterocyclic. Polycyclic unsaturated compounds may also include compounds having fused rings.

Optionally, the unsaturated compound is selected from the group comprising, but not limited to, molecules **1a** (1,3-diphenyl-propenone); **1b** (3-(4-methoxy-phenyl)-1-phenyl-propenone); **1c** (3-(4-nitro-phenyl)-1-phenyl-propenone); **3a** (3-methyl-4-nitro-5-styryl-isoxazole); **3b** (5-[2-(4-methoxy-phenyl)-vinyl]-3-methyl-4-nitro-isoxazole); **3c** (3-methyl-4-nitro-5-[2-(4-nitro-phenyl)-vinyl]-isoxazole); **3d** (5-[2-(4-fluoro-phenyl)-vinyl]-3-methyl-4-nitro-isoxazole); **3e** (5-[2-(4-chloro-phenyl)-vinyl]-3-methyl-4-nitro-isoxazole); **5** ((2-nitro-vinyl)-benzene); **7** (but-3-en-2-one); **9** (4-methyl-pent-3-en-2-one); **11** (3-phenyl-acrylic acid ethyl ester); **13** ([1,2]naphthoquinone); **15** ([1,4]naphthoquinone); **17** (cyclohex-2-enone); and ); and **23** (3-phenyl-acrylonitrile) of Table 2.

By the term "cyclic derivatives of alkenes" is meant an epoxide or an aziridine. Optionally the epoxide is linear or branched. Optionally the epoxide is monosubstituted, disubstituted, trisubstituted or tetrasubstituted. One suitable epoxide is styrene oxide. Optionally the aziridine is monosubstituted, disubstituted, trisubstituted or tetrasubstituted. Optionally the nitrogen of the aziridine is substituted with one or more acyl, aryl or alkyl groups. Preferably, the nitrogen of the aziridine is non substituted (H).

Without being bound by theory, it is thought that the likely reaction mechanism is set forth below:

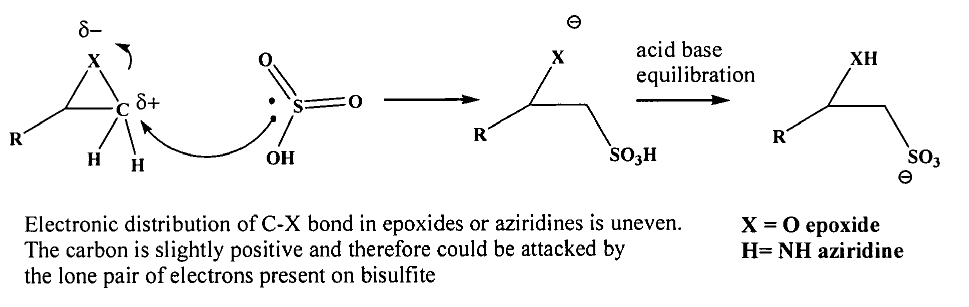

### Catalyst

The catalyst capable of activating the sulfur having at least one lone pair of electrons acts both to deprotonate the donor compound having at least one sulfur having at least one lone pair of electrons, so that the donor compound then becomes nucleophilic enough to attack the acceptor compound and, additionally, to impart an acceleration to the reaction. The acceleration is carried out by forming a salt with the donor compound, as will be exemplified hereunder. In this context, formation of specific hydrogen bond activates the donor compound to nucleophilic addition. It is thought that the proton in bisulfite is located on the sulfur (structure B below). However, structure B is in equilibrium with structure A (also below), which is the reactive species in the sulfonylation reaction observed. The catalyst (amine in certain embodiments) is able to stabilise the most reactive species A that is present in increased amount. The enhanced concentration of species A leads to an enhanced reaction rate, producing a net activation of bisulfite. 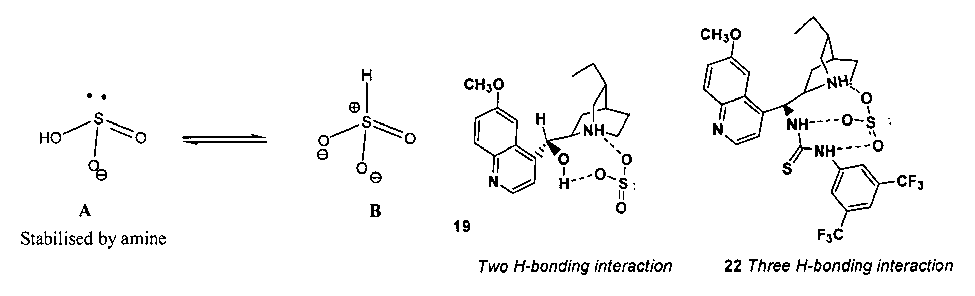 By the term "activating" is meant stabilising the sulfonylation reactive species, possibly by hydrogen bond interactions with the catalyst (amine in certain embodiments).

Preferably, the catalyst is a nucleophile.

Optionally, the nucleophile comprises at least one valence electron that does not form part of a covalent bond. Preferably, the nucleophile comprises at least one pair of valence electrons, wherein the at least one pair of electrons does not form part of a covalent bond.

Preferably, the nucleophile is an amine.

Preferably, the nucleophile comprises one or more hydrogen bond acceptors, and at least one hydrogen bond donor. Optionally, the nucleophile comprises one or two hydrogen bond acceptors, and one or two hydrogen bond donors. By the term "hydrogen bonds" is meant electrostatic attractions between a hydrogen bearing a partial, positive charge and another atom (usually O or N) bearing a partial negative charge. These partial opposite charges are a consequence of the relative electronegativity of covalently-bonded atoms. By the term "hydrogen bond donor" is meant a molecule containing an hydrogen bound to an electronegative element (N, O, S, for example). By the term "hydrogen bond acceptor" is meant a molecule containing atoms having localised non bonding electron pairs (lone pair).

Optionally, the amine is a cyclic amine. Further optionally, the amine is a heterocyclic amine.

Optionally, the amine is pyridine.

Preferably, the amine is an aliphatic amine.

Optionally, the amine is a secondary amine.

Preferably, the amine is a tertiary amine. Optionally, the amine comprises at least one nitrogen atom having at least one pair of valence electrons, wherein the at least one pair of electrons does not form part of a covalent bond; and at least three functional groups. Each functional group may be the same functional group or a different functional group. Preferably, each of the three functional groups is a different functional group. Preferably, the amine is triethylamine.

Without being bound by theory, pyridine is an aromatic amine while triethylamine is not, therefore the lone pair in pyridine is delocalised and is thought to be less available to engage in hydrogen-bonding (acceptor). In triethylamine, the lone pair is present stably on the nitrogen and therefore a more tight pair could be formed with bisulfite.

Optionally, the reaction is carried out at between -20 and 35°C, optionally either between - 20°C and 5°C or between 18 and 22°C.

Optionally, the reaction is carried out for a period of time such that an isolatable amount of the sulfur-containing compound is produced. Optionally, the period of time is between 20min and 24h, optionally between 1h and 20h.

Optionally, 0.01 equiv to 10 equiv of catalyst is provided, per equiv of the donor compound. By the term "equiv" is meant molar equivalents. For example, 0.05 equiv to 10 equiv, optionally 0.05 equiv to 5 equiv, further optionally 0.05 equiv to 1 equiv, still further optionally 0.05 equiv to 0.5 equiv, of catalyst is provided, per equiv of the donor compound.

In a preferred embodiment of the present invention, there is provided a method for preparing heterochiral sulfur-containing compounds, in which the method comprises reacting
a donor compound comprising at least one sulfur having at least one lone pair of electrons with
an acceptor compound;
wherein the reaction occurs in the presence of a chiral catalyst capable of activating the sulfur having at least one lone pair of electrons.

Preferably, the chiral catalyst is a chiral amine. Further preferably, the chiral amine is selected from the group comprising, but not limited to, molecules **19** ((5-ethyl-1-aza-bicyclo[2.2.2]oct-2-yl)-(6-methoxy-quinolin-4-yl)-methanol); **19a** (1-(3,5-bis-trifluoromethyl-phenyl)-3-[(5-ethyl-1-aza-bicyclo[2.2.2]oct-2-yl)-(6-methoxy-quinolin-4-yl)-methyl]-thiourea); **20** (pyrrolidin-2-yl-methanol); and **21** (2-amino-2-phenyl-ethanol) of Schemes 3 and 4 hereunder.

By the term "heterochiral" is meant a chiral compound having an enantiomeric species
wherein one enantiomeric form (R or S) is in excess of the other.

Optionally, the sulfur-containing compounds are substantially homochiral. By substantially homochiral is meant an enantiomeric species comprising more than 85%, optionally more than 90%, further optionally more than 95%, still further optionally more than 99%, of one enantiomeric form (R or S).

Without being bound by theory, it is thought that lowering the reaction temperature to 5°C or below may increase the enantiomeric excess. Indeed, Example 3 hereunder demonstrates that lowering the reaction temperature to 0 - 5°C from room temperature does improve the enantiomeric excess.

In addition, it is thought that the dielectric constant of the reaction solvent may influence the enantiomeric excess obtained. Suitable aprotic reaction solvents include, but are not limited to, tetrahydrofuran (close to 0), hexane (1.9), dioxane (2.2), chloroform (4.8), acetonitrile (37) and dimethyl sulfoxide (47). Suitable protic solvents include, but are not limited to, butanol (12.5), ethanol (24.5), methanol (32.7), of which methanol is preferred.

Without being bound by theory, it is thought that ionic couples form more quickly in polar solvents, as the polar ions have to travel around the media to find each other. However, ionic couples are thought to be more stable in aprotic and apolar solvents. The optimal conditions arise from combination of ease of formation and stability of the salt, which, in our case, occurred in methanol.

Further optionally, the sulfur-containing compounds are homochiral. By homochiral is meant an enantiomeric species comprising only one enantiomeric form (R or S).

Preferably, the reaction is enantiospecific. By "enantiospecific" is meant capable of synthesising a product having high enantiomeric purity, i.e. that a product comprising a single enantiomer is synthesised. However, it is understood that the product does not have to be exclusively enantiopure, but may be partially enantiopure, and that enantioselective capabilities also fall within the scope of this definition.

Optionally, the reaction is regiospecific. By "regiospecific" is meant being capable of synthesising a product in which one structural isomer is produced in favour of other isomers are also theoretically possible. However, it is understood that the given structural isomer product does not have to be exclusively synthesised, but other structural isomers may be synthesised, and that regioselective capabilities also fall within the scope of this definition.

Optionally, the reaction is stereospecific. By "stereospecific" is meant capable of synthesising products having the same atomic connectivity, wherein a product having a given atomic arrangement in space, or structural configuration, is synthesized in favour of other products of different atomic arrangement in space. However, it is understood that the product having a given atomic arrangement in space does not have to be exclusively synthesised, but products with other structural configuration may be synthesised, and that stereoselective capabilities also fall within the scope of this definition.

Optionally, the reaction is carried out at between -20 and 35°C, optionally between -20 and 5°C.

Optionally, the reaction is carried out for a period of time such that an isolatable amount of the sulphur-containing compound is produced. Optionally, the period of time is between 20min and 24h, optionally between 1h and 20h.

The mild conditions adopted in the methods of the present invention allow the preparation of sulfur-containing compounds that contain heat and/or radical sensitive functionalities. As a result, sulfur-containing compounds have been prepared that could not be obtained before using the existing methodology.

Accordingly in a second aspect of the invention, there is provided sulfur-containing compounds of the formula: 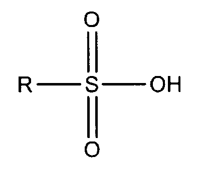 wherein R is selected from:
(a) 1-(4-Nitro-phenyl)-3-oxo-3-phenyl-propane;
(b) 2-(3-Methyl-4-nitro-isoxazol-5-yl)-I-phenyl-ethane;
(c) 1-(4-Methoxy-phenyl)-2-(3-methyl-4-nitro-isoxazol-5-yl)-ethane;
(d) 2-(3-Methyl-4-nitro-isoxazol-5-yl)-1-(4-nitro-phenyl)-ethane;
(e) 1-(4-Fluoro-phenyl)-2-(3-methyl-4-nitro-isoxazol-5-yl)-ethane;
(f) 1-(4-Chloro-phenyl)-2-(3-methyl-4-nitro-isoxazol-5-yl)-ethane;
(g) 3-Oxo-butane;
(h) 2-Methyl-4-oxo-pentane-2-yl;
(i) 3,4-Dihydroxy-naphthalene-2-yl; and
(j) 3-Oxo-cyclohexane.

In a third aspect of the invention, there is provided use of chiral sulfur-containing compounds obtainable by the method of the first aspect of the invention or chiral sulfur-containing compounds of the second aspect of the invention for the resolution of racemic mixtures of amines. Families of sulfonic acids such as **4a-e** could be used to resolve racemic amines (Dutch Resolution). The use of structurally related (families) sulfonic acids has been proved an efficient method to separate two amine enantiomers. However, this methodology is hampered by the limited number of families of homochiral sulfonic acids.

The method of the present invention allows the preparation of families of enantiopure sulfonic acids, for example, **4a-e** or **2a-c.**

It will be appreciated that sulfonic acids prepared in accordance with the method of the present invention can used as surfactants, as synthetic intermediates and / or as resolving agents.

### Materials and Methods

### Preparation of Compounds (Example 1)

To a solution of alkene (2.17 mmol) in THF (10 ml) was added aqueous NaHSO₃ (38 %) solution (5.95 mL, 21.7 mmol) followed by triethylamine (3 mL, 21.7 mmol) at room temperature (RT), and stirred for the number of hours indicated in Table 1. After this time, the salts were filtered, the organic layer separated and evaporated to give the triethylammonium salt of the product in pure form. The salt was then dissolved in water (10 ml) and passed through a DOWEX™ acidic ion exchange resin to eliminate triethylamine. Evaporation of the aqueous layer under reduced pressure at 50°C gave pure sulfinic acid ester, as a colourless solid.

### Preparation of Compounds (Example 2)

To a solution of alkene (250mg, 1.0 mmol) in THF (5 ml) was added aqueous NaHSO₃ (38 %) solution (2.47 ml, 10 mmol) followed by TEA (triethylamine) (1.4 ml, 10 mmol) at room temperature, and stirred for 1h. After this time, the salts were filtered, the organic layer separated and evaporated to give the triethylammonium salt of the product in pure form. The salt was then dissolved in water (10 ml) and passed through a DOWEX™ acidic ion exchange resin to eliminate triethylamine. Evaporation of the aqueous layer under reduced pressure at 50°C gave pure sulfinic acid ester, as a colourless solid.

### Examples

The following examples are described herein so as to provide those of ordinary skill in the art with a complete disclosure and description of the invention, and are intended to be purely exemplary of the present invention, and are not intended to limit the scope of the invention.

### Example 1. Preparation of sulfonic acid

During our studies on the dihydroxylation of alkene **3** [Adamo, M. F. A. et Al., Org. Lett. 2008, 10, 1807], we noticed, but did not report, that remainder starting material **3** reacted with sodium bisulfite during work up. This reaction occurred only when an excess of pyridine was present (**Table 1**). Pyridine was used as the solvent and must be used in at least 5 mL per mmol. When used in such a large excess, pyridine gives good results.

The reaction with "diluted" pyridine [pyridine/THF, first entry below] is slower (requires 12h) and gives 60% yield, whilst the "undiluted reaction"[pyridine as solvent, second entry below] requires 3-4 h and gives higher yields. No reaction occurs in the absence of pyridine [last entry below].

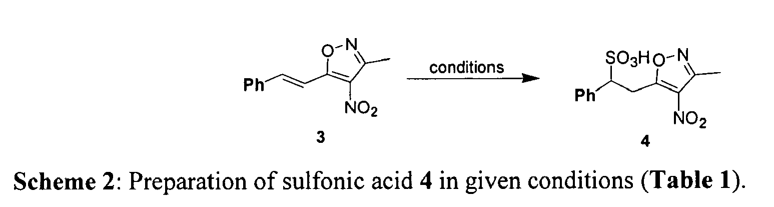

**Table 1: Yields of sulfonic acid 4.**

| **Reaction conditions^{a}** | **Time (hours)** | **Yields of 4^{b}** |
|---|---|---|
| Pyridine / THF, NaHSO₃ | 12h | 60% |
| Pyridine, NaHSO₃ | 2-3h | 75% |
| THF, NaHS0₃ | 12h | 0% |

| | | |
|---|---|---|
| ^{a} reaction conditions: ^{b} yields after flash chromatography, room temperature | | |

TEA, as used in Example 2 below, is thought to be a superior catalyst compared to pyridine, as used herein, because TEA is more capable of engaging in hydrogen-bonding with bisulfite. It is thought that the tighter pair leads to higher reactivity.

### Example 2. Preparation of sulfonic acid using different alkenes.

Several alkenes have been submitted to reaction with sodium bisulfite in the presence of pyridine, in first instance, and simple amines such as triethylamine (TEA).

Triethylamine and tertiary amines in general showed to be superior catalysts compared to pyridine and were used in limited amounts. Pyridine is an aromatic amine while triethylamine is not, therefore the lone pair in pyridine is delocalised and less available to engage in hydrogen-bonding (acceptor). In triethylamine, the lone pair is present stably on the nitrogen and therefore a tighter pair could be formed with bisulfite.

Representative results are summarised in **Table 2.** The reactions in **Table 2** were carried out at room temperature (RT) in a very short time, in THF as the reaction solvent and without the aid of radical initiators.

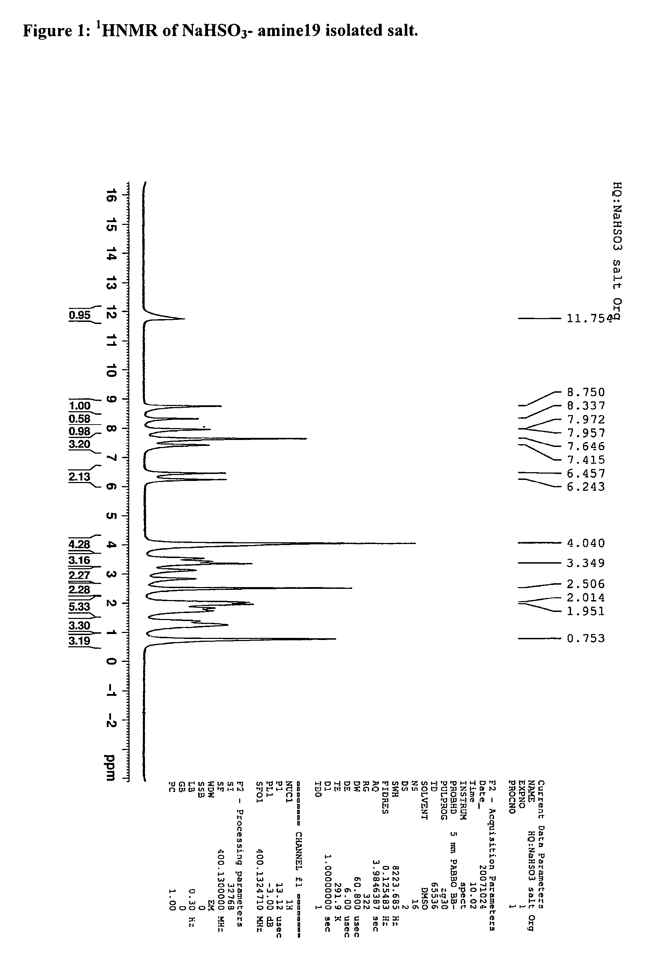

We have briefly studied the reaction mechanism and established the following facts:
(a) The conversion of **1** to **2** occurred in the absence of light and in the presence of radical scavengers, demonstrating that sulfonic acid **2** arose from thia-Michael addition of bisulfite to the alkene.
(b) reaction of bisulfite with the amine produce a solid salt that could be isolated (see Figure 1 of the accompanying drawings). Treatment of the alkene with the salt produced the desired sulfonic acid.

**Table 2**

| **substrate** | **Amount of NaHSO₃** | **Amount of TEA** | **Time (hours) and temperature (RT is room temperature)** | **Product** | **Yield %** |
|---|---|---|---|---|---|
| 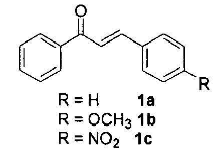 | 10mol eq | 10mol eq | 1h RT | 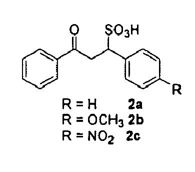 | **2a** 93 |
| | | | | | **2b** 98 |
| | | | | | **2c** 94 |
| 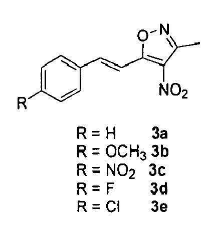 | 10mol eq | 10mol eq | 1h RT | 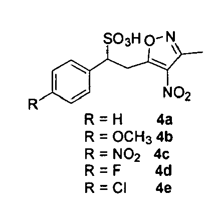 | **4a** 75 |
| | | | | | **4b** 80 |
| | | | | | **4c** 82 |
| | | | | | **4d** 86 |
| | | | | | **4e** 81 |
| 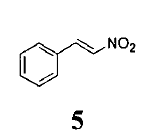 | 10mol eq | 10 mol eq | 1h RT | 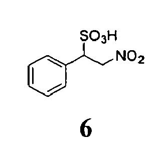 | 92 |
| 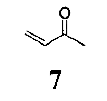 | 1.2mol eq | 1.2mol eq | 2h RT | 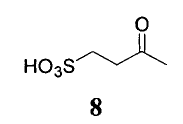 | 88 |
| 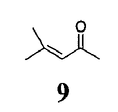 | 1.2mol eq | 1.2mol eq | 16h RT | 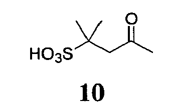 | 82 |
| 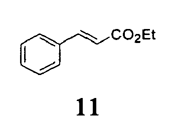 | 20mol eq | 20mol eq | 14h RT | 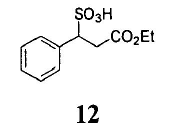 | 71 |
| 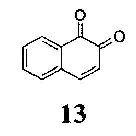 | 10mol eq | 10mol eq | 4h RT | 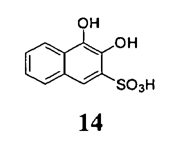 | 79 |
| 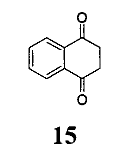 | 10mol eq | 10mol eq | 4h RT | 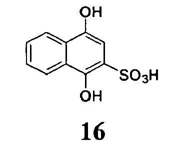 | 84 |
| 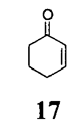 | 1.2mol eq | 1.2mol eq | 2h RT | 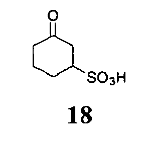 | 91 |
| 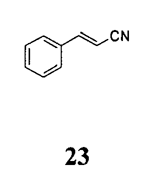 | 20mol eq | 20mol eq | 14h RT | 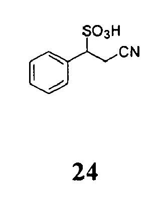 | 89 |
| **23** 3-Phenyl-acrylonitrile | | | | | |
| **24** 2-Cyano-1-phenyl-ethanesulfonic acid | | | | | |

### Example 3. Preparation of sulfonic acid in enantiomeric excess.

The reaction of **1** with bisulfite in the presence of 10 equiv of enantiopure hydroquinine **19** and 10 equiv of sodium bisulfite produced **2** in 84% isolated yields and in 33% enantiomeric excess. The use of amines **20** and **21** (illustrated in Scheme 3 below) furnished **2** in similar yields and in 11-15% enantiomeric excess. Importantly, in these experiments, the amine was recovered at the end of the reaction. To date, this is the unique example of asymmetric sulfonylation observed. 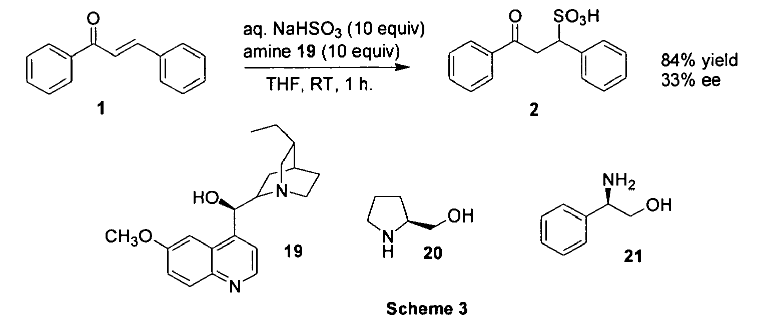 To a solution of chiral amine (1.1 mmol) in MeOH (10 mL) was added aq. NaHSO₃ (38 %) solution dropwise (0.32 mL, 1.1 mmol). The solution was stirred for one hour at room temperature, then cooled to 0-5°C and stirred for 30 minutes, treated with a solution of alkene (1.0 equiv) in THF (10 mL). The reaction mixture was then stirred for 12 hours at room temperature. After this time, the solvents were evaporated to dryness, the solid obtained were treated with chloroform (15 mL) and filtered. The chloroform layer was evaporated and the crude was analysed by ¹H-NMR and weighed.

Catalyst **19a** could be used in just 1.1 equiv ensuring 100% conversion of alkene **1** and over 99% yield of desired **2.** Importantly, catalyst **19a** allowed the preparation of sulfonic acid **2** in 86% enantiomeric excess **(Scheme 4** and **Table 3**).

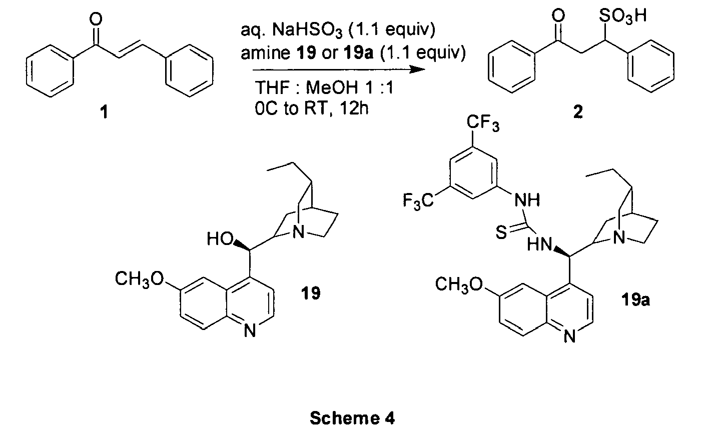

**Table 3**

| **amine** | **NaHSO₃** | **Yields% 2** | **ee%** |
|---|---|---|---|
| NEt₃ (1.1 equiv) | 1.1 equiv | 30% | 0 |
| **19** (1.1 equiv) | 1.1 equiv | 50% | 30% |
| **19a** (1.1 equiv) | 1.1 equiv | >99% | 86% |

Without being bound by theory, we postulate that activation of bisulfite occurs through formation of H-bonds. We also have proposed that the higher turnover of catalyst **25** (**Scheme 5**) over triethylamine, depends on the ability of compound **25** to form two H-bonds with bisulfite, while triethylamine could make only one such H-bond.

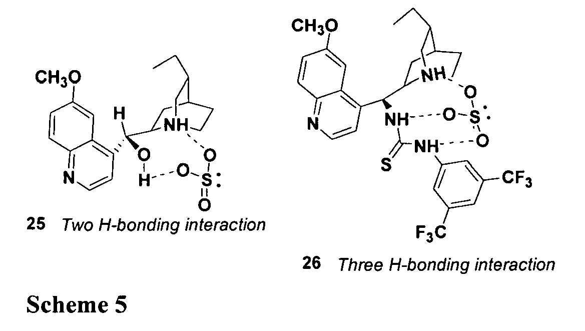

It is reasoned that the use of catalysts capable of three H-bonds, such as thiourea **26,** should impart to bisulfite, enhanced reactivity (**Scheme 5**). The concept was proved true and the catalyst **26** could be used in just 1.1 equiv, ensuring 100% conversion of alkene **1a-c** and over 99% yield of the desired **2a-c.** Importantly, catalyst **26** allowed the preparation of sulfonic acid **2a-c** in 86-88% enantiomeric excess.

The NaHSO₃- amine isolated salt (intermediate) **22,** in which the bisulfite is in hydrogen bonding interaction with catalyst **19,** is characterised by ¹HNMR in Figure 1 of the accompanying drawings.

### Example 4: Procedure for the addition of bisulfite to epoxides or aziridines

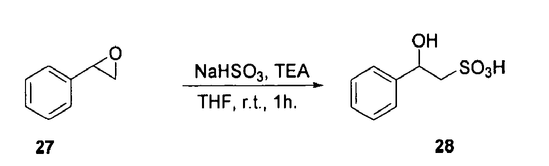

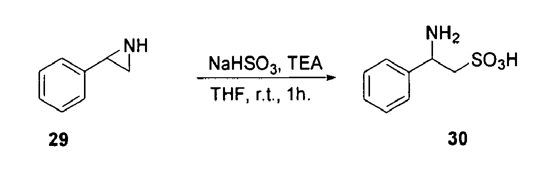

**27** 2-Phenyl-oxirane
**28** 2-Hydroxy-2-phenyl-ethanesulfonic acid
**29** 2-Phenyl-aziridine
**30** 2-Amino-2-phenyl-ethanesulfonic acid

To a solution of amine (1.1 mmol) in MeOH (10 mL) was added aq. NaHSO₃ (38 %) solution dropwise (0.32 mL, 1.1 mmol). The solution was stirred for one hour at room temperature, then cooled to 0-5°C and stirred for 30 minutes, treated with a solution of epoxide 26 or aziridine 28 (1.0 equiv) in THF (10 mL). The reaction mixture was then stirred for 12 hours at room temperature. After this time, the solvents were evaporated to dryness, the solid obtained were treated with chloroform (15 mL) and filtered. The chloroform layer was evaporated and the crude was analysed by ¹H-NMR and weighed.
Yield for **28** 169 mg, 84% yield
Yield for **30** 163 mg, 81 % yield

The invention is not limited to the embodiments described herein but can be amended or modified without departing from the scope of the present invention.

## Claims

1. A method for preparing sulfur-containing compounds, the method comprising reacting
a donor compound comprising at least one sulfur having at least one lone pair of electrons,
with an acceptor compound;
wherein the reaction occurs in the presence of a catalyst capable of activating the sulfur having at least one lone pair of electrons.

2. A method according to Claim 1, in which the sulfur-containing compounds include sulfonic acids, sulfinic acids, sulfenic acids, and esters thereof or amides thereof, sulfones and sulfoxides.

3. A method according to Claim 1 or 2, in which the reaction occurs in the presence of a reaction solvent selected from the group consisting of dimethyl sulfoxide, dimethylformamide, dioxane and hexamethylphosphorotriamide and tetrahydrofuran; in which, optionally, the reaction solvent is tetrahydrofuran or, alternatively, a reaction solvent selected from the group consisting of butanol, ethanol and methanol, of which methanol is preferred.

4. A method according to any one of Claims 1 - 3, in which the at least one sulfur having at least one lone pair of electrons is capable of being transferred to the acceptor compound, either via the formation of an intermediate compound, or directly to the acceptor compound.

5. A method according to Claim 4, in which the at least one sulfur having at least one lone pair of electrons is a sulfite anion.

6. A method according to any one of Claims 1 - 5, in which the donor compound is sodium hydrogen sulfite (sodium bisulfite).

7. A method according to any one of Claims 1 - 6, in which the acceptor compound comprises an unsaturated compound or a cyclic derivative thereof, the cyclic derivative being optionally selected from an epoxide or an aziridine.

8. A method according to Claim 7, in which the acceptor compound comprises an unsaturated compound, the unsaturated compound having at least one electron-withdrawing group is adjacent the at least one multiple bond.

9. A method as claimed in Claim 8, in which the electron-withdrawing group is selected from the group consisting of carbonyl groups, and their esters; nitro groups; cyano groups; oximes, hydrazones, imino groups; protected imino groups such as BOC or CBZ protected imines.

10. A method according to any one of Claims 1 - 9, in which the catalyst is a nucleophile comprising at least one valence electron that does not form part of a covalent bond.

11. A method as claimed in Claim 10, in which the nucleophile comprises at least one hydrogen bond acceptor, and at least one hydrogen bond donor.

12. A method as claimed in Claim 10 or 11, in which the nucleophile is an amine.

13. A method according to any one of Claims 1 - 12, in which the catalyst is a chiral amine, optionally selected from the group comprising molecules **19** ((5-ethyl-1-aza-bicyclo[2.2.2]oct-2-yl)-(6-methoxy-quinolin-4-yl)-methanol); **19a** (1-(3,5-bis-trifluoromethyl-phenyl)-3-[(5-ethyl-1-aza-bicyclo[2.2.2]oct-2-yl)-(6-methoxy-quinolin-4-yl)-methyl]-thiourea); **20** (pyrrolidin-2-yl-methanol); and **21** (2-amino-2-phenyl-ethanol).

14. Sulfur-containing compounds of the formula: 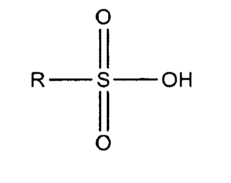 wherein R is selected from:
(a) 1 -(4-Nitro-phenyl)-3-oxo-3-phenyl-propane;
(b) 2-(3-Methyl-4-nitro-isoxazol-5-yl)-1-phenyl-ethane;
(c) 1-(4-Methoxy-phenyl)-2-(3-methyl-4-nitro-isoxazol-5-yl)-ethane;
(d) 2-(3-Methyl-4-nitro-isoxazol-5-yl)-1-(4-nitro-phenyl)-ethane;
(e) 1-(4-Fluoro-phenyl)-2-(3-methyl-4-nitro-isoxazol-5-yl)-ethane;
(f) 1-(4-Chloro-phenyl)-2-(3-methyl-4-nitro-isoxazol-5-yl)-ethane;
(g) 3-Oxo-butane;
(h) 2-Methyl-4-oxo-pentane-2-yl;
(i) 3,4-Dihydroxy-naphthalene-2-yl; and
(j) 3-Oxo-cyclohexane.

15. Use of chiral sulfur-containing compounds obtainable by the method according to any one of Claims 1 - 13 or chiral sulfur-containing compounds according to Claim 14 for the resolution of racemic mixtures of amines.
